## Europäisches Patentamt
## European Patent Office
## Office européen des brevets

(19)

(11) Numéro de publication: **0 099 780 B1**

(12) **FASCICULE DE BREVET EUROPEEN**

(45) Date de publication du fascicule du brevet:
**06.09.89**

(51) Int. Cl.⁴: **C 08 H 1/06, A 61 K 7/06**

(21) Numéro de dépôt: **83401305.4**

(22) Date de dépôt: **23.06.83**

(54) Dérivé kératinique, son procédé de préparation et composition de traitement le contenant.

(30) Priorité: **24.06.82 FR 8211085**

(43) Date de publication de la demande:
**01.02.84 Bulletin 84/5**

(45) Mention de la délivrance du brevet:
**06.09.89 Bulletin 89/36**

(84) Etats contractants désignés:
**AT BE CH DE FR GB IT LI NL SE**

(56) Documents cités:
**US-A- 3 033 755**
**US-A- 3 660 566**

**NATURE, vol. 225, no. 5237, 14 mars 1970, pages 1052-1053, MacMillan (Journals) Ltd., USA L.A. GOLDSMITH et al.: "Uniquely oriented epidermal lipid"**

Le dossier contient des informations techniques présentées postérieurement au dépôt de la demande et ne figurant pas dans le présent fascicule.

(73) Titulaire: **L'OREAL, 14, Rue Royale, F-75008 Paris (FR)**

(72) Inventeur: **Brod, Joel, 89, rue de l'Ourcq, F-75019 Paris (FR)**
Inventeur: **Kermici, Michel, 36, rue de Picpus, F-75012 Paris (FR)**
Inventeur: **Schaefer, Hans, Villa La Bergerie Chemin de la Constance, F-06600 Antibes (FR)**

(74) Mandataire: **Peuscet, Jacques, Cabinet Peuscet 68, rue d'Hauteville, F-75010 Paris (FR)**

## Description

La présente invention a trait à un dérivé kératinique extrait d'une matière première d'origine animale, éventuellement chimiquement modifié, ce dérivé ayant subi un traitement agissant sur les molécules liposolubles contenues dans la matière première. La présente invention vise aussi l'obtention d'un tel dérivé, et les compositions de traitement d'un substrat kératinique, tel que la peau ou les cheveux humains, ces compositions contenant le dérivé kératinique susmentionné.

On sait que la peau a une structure dans laquelle la répartition des lipides varie, quand on se déplace du derme vers le stratum cornéum. Dans la zone de la couche granuleuse, les espaces intercellulaires contiennent, approximativement, autant de lipides polaires que de lipides non-polaires (lipides neutres); quand on s'éloigne de la couche granuleuse, on note une disparition progressive des lipides polaires et, au niveau des cornéocytes du stratum cornéum, les espaces intercellulaires ne contiennent plus que 15% de lipides polaires (par rapport aux lipides totaux). Le caractère hydrophobe de la couche superficielle de la peau résulte de la prépondérance des lipides non-polaires (lipides neutres) dans les espaces intercellulaires du stratum cornéum. Si l'on désire faciliter la pénétration d'un composé à travers la peau, on a intérêt à augmenter la quantité de lipides polaires dans la couche superficielle; si, au contraire, on désire réduire les échanges à travers la peau, par exemple réduire la perte insensible en eau dans le sens derme-épiderme, il convient d'augmenter la proportion de lipides non-polaires (lipides neutres) dans la couche superficielle.

L'invention a pour but de décrire un dérivé kératinique constitué d'un polymère kératinique extrait des cellules d'un matériau kératinique d'origine animale, ledit polymère ayant un caractère lipidique modifié par rapport à celui qu'il avait dans la matière première de départ. L'invention concerne aussi une composition, qui permet d'agir de façon efficace sur la teneur en molécules liposolubles de la couche superficielle d'un substrat kératinique, tel que la peau ou les cheveux humains. Cette composition permet donc de faire varier, selon le but recherché, la possibilité de traversée de la couche externe du substrat par un produit; par exemple, on pourra chercher à extraire du substrat kératinique une substance qui s'y trouve ou, au contraire, faire pénétrer dans le substrat kératinique une substance que l'on apporte de l'extérieur. Selon l'invention, on agit sur la teneur en molécules liposolubles du substrat kératinique en mettant en contact avec ce substrat un dérivé kératinique, dont l'état de charge en molécules liposolubles est différent de celui qui existe naturellement dans la kératine. On a, en effet, constaté, selon l'invention, que l'on pouvait, à partir d'une matière première kératinique, chimiquement modifiée ou non, extraire une grande partie, ou même la totalité des lipides naturellement contenus dans la matière kératinique et que l'on pou

vait également, sur une telle matière kératinique délipidée, réassocier des molécules liposolubles de chargement éventuellement différentes de celles que l'on a extraites au cours de la dépilation et/ou modifier chimiquement la matière kératinique délipidée ou rechargée en liposoluble(s). On a, par ailleurs, constaté que l'on pouvait fixer sur une matière première kératinique non-délipidée un excès de molécules liposolubles de chargement. On voit donc que, selon l'invention, on peut obtenir ou bien un dérivé kératinique sous-chargé en molécules liposolubles (délipidation totale comprise), ou bien un dérivé kératinique surchargé en molécules liposolubles, la nature des molécules liposolubles associées aux chaînes kératiniques pouvant être, grâce au choix de molécules de chargement appropriées, complètement modifiée par rapport à la nature des molécules liposolubles se trouvant dans la matière première de départ.

Dans la présente description et dans les revendications associées, on utilise l'expression «molécules liposolubles» (ou encore «liposolubles») pour désigner de façon générique:

a) des composés insolubles dans l'eau mais solubles dans des solvants polaires ou non-polaires, pris isolément ou en mélange; dans cette catégorie se trouvent notamment les lipides tels que définis par la classification de LEHNINGER (LEHNINGER-Editions Flammarion – 2e édition, chapitre 11, pages 275–303); ces lipides peuvent être classés en saponifiables et insaponifiables: parmi les lipides insaponifiables, on peut citer les terpènes (polymères de l'isoprène), les vitamines liposolubles, par exemple A, E, F et K, et les stéroïdes, par exemple l'hydrocortisone et les oestrogènes;

b) des composés, solubles dans un ou plusieurs lipides, pouvant être associés à la kératine, seuls, ou solubilisés dans un ou plusieurs lipides: dans cette catégorie se trouvent, par exemple, des colorants liposolubles, des humectants liposolubles, des filtres solaires liposolubles, ou encore l'acide rétinoïque.

Selon l'invention, on a constaté que si l'on mettait en contact un dérivé kératinique à caractère lipidique modifié avec un substrat kératinique, tel que la peau ou les cheveux humains, on pouvait obtenir une modification du substrat kératinique traité et de ses caractéristiques. Si on utilise, par exemple, un dérivé kératinique riche en lipides polaires, on facilite la pénétration dans la peau de produits amenés de l'extérieur, par exemple de produits de traitement hydrophiles. Au contraire, si l'on applique des dérivés kératiniques chargés en lipides non-polaires (lipides neutres), on réduit le risque de pénétration dans la peau à partir de l'extérieur et on réduit la perte insensible en eau du sujet traité.

Si l'on utilise un dérivé kératinique sous-chargé en lipides, les lipides du substrat kératinique ont tendance à aller s'associer aux chaînes kératiniques du dérivé kératinique mis en contact avec le substrat, ce qui permet d'avoir un effet dégraissant: on peut, de la sorte, réduire la quantité de sébum sur la peau ou sur les cheveux, les dérivés kératiniques utilisés se comportant comme une éponge à sébum. On peut envisager également d'utiliser des dérivés kératiniques selon l'invention pour protéger le cuir chevelu lors d'un traitement des cheveux par des produits hydrophiles, dont on cherche à limiter le risque de pénétration dans la peau: dans ce cas, on utilise les dérivés kératiniques surchargés en lipides non-polaires (lipides neutres).

Selon l'invention, on a constaté également qu'un dérivé kératinique surchargé en liposolubles, contenant par exemple de 30 à 70% en poids de liposolubles par rapport au poids total du dérivé, se présente dans l'eau sous forme d'une émulsion stable: on constate donc que la fraction d'origine kératinique du dérivé a des propriétés émulsifiantes.

Dans l'état de la technique, on a déjà proposé d'utiliser des produits d'origine kératinique ayant subi une délipidation préalable: c'est le cas notamment des produits définis dans le brevet allemand 556 488 et dans les brevets des Etats Unis d'Amérique 3 033 755 et 3 660 566. Cependant, dans tous les cas, le traitement du matériau laisse subsister les cellules: les chaînes polymériques kératiniques, qui constituent les filaments kératiniques intracellulaires, ne sont pas libérées. Il en résulte que les produits antérieurement décrits ne sont pas, contrairement aux dérivés selon l'invention, constitués de polymères kératiniques à l'état libre et n'ont donc pas le même comportement pour le traitement d'un substrat kératinique. Cette différence est due non seulement au fait que les chaînes kératiniques sont englobées dans des cellules fermées au lieu d'être à l'état de polymère libre, mais encore au fait que les chaînes kératiniques constitutives du dérivé selon l'invention sont, dans ce dérivé, à une concentration beaucoup plus forte que dans le produit cellulaire de l'état de la technique, qui contient les ciments extra-cellulaires, les protéines non kératiniques intra-cellulaires et les membranes cellulaires. On a aussi prévu, notamment dans le brevet américain 3 660 566 précité, de relipider au moins partiellement le produit cellulaire délipidé: mais une telle relipidation s'effectue différemment de celle que l'on propose selon l'invention: en effet, dans l'état de la technique, les lipides de rechargement reconstituent, pour une large part, les ciments extra-cellulaires et les membranes; ils ne servent donc que peu à la formation de complexes avec les chaînes polymériques hélicoïdales de la kératine. Au contraire, selon l'invention, le rechargement en liposoluble(s) après délipidation affecte directement les chaînes polymériques de la kératine et le dérivé obtenu est donc différent du produit antérieurement décrit. En outre, le polymère rechargé en liposoluble(s) de l'invention est à l'état libre et non englobé dans des cellules de la matière première.

La présente invention a donc pour objet un dérivé kératinique provenant d'une matière première contenant au moins une kératine d'origine animale, caractérisé par le fait qu'il est constitué par un polymère kératinique obtenu par séparation de substances kératiniques intracellulaires avec élimination des parois cellulaires, ledit polymère étant au moins partiellement délipidé.

La matière première animale utilisée peut avoir ou non, avant l'éventuel chargement en liposoluble(s), subi un traitement chimique modifiant le squelette kératinique tel que, par exemple, une hydrolyse et/ou une oxydation fournissant des groupes sulfoniques et/ou une carboxyalkylation.

Comme liposolubles de chargement on peut citer, à titre d'exemples, les colorants liposolubles, les humectants liposolubles, les filtres solaires liposolubles, les vitamines liposolubles, les stérols, les acides gras, les esters d'acide gras, les lipides phosphorylés, les sphingolipides, l'acide rétinoïque.

Dans la suite de la présente description et des revendications associées, on désignera par $T_n$ le taux pondéral de lipides dans la kératine native servant de matière première; par $T_i$ le taux pondéral de liposolubles d'un dérivé kératinique selon l'invention sous-chargé en liposolubles; par $T_s$ le taux pondéral de liposolubles dans un dérivé kératinique selon l'invention sur-chargé en liposolubles; et par $T_c$ le taux pondéral de(s) liposoluble(s) de chargement ajouté(s) à la matière kératinique après qu'elle ait ou non subi préalablement un traitement de délipidation totale ou partielle.

Dans une première variante, le dérivé kératinique est un polymère ne contenant plus aucun lipide associé: ce polymère résulte d'une délipidation totale de la matière première utilisée et, dans ce cas, le taux $T_i$ est nul ou sensiblement nul.

Dans une deuxième variante, le dérivé kératinique est un polymère associé à un ou plusieurs liposoluble(s) à un taux $T_i$ non nul: on a $T_i < T_n$. Dans cette variante, le dérivé kératinique est donc sous-chargé en liposoluble(s) par rapport à la matière première de départ, ce(s) liposoluble(s) pouvant être ou non ceux qui figuraient dans la matière première de départ. Lorsque le(s) liposoluble(s) est (ou sont) différent(s) de celui (ou ceux) figurant dans la matière première de départ, le dérivé kératinique selon l'invention résulte d'une première étape de délipidation totale ou partielle réalisée sur la matière première utilisée jusqu'à un taux $t_i$. Si aucune étape de chargement n'intervient ensuite, on a $T_i = t_i$. Si, au contraire, on charge la matière délipidée avec au moins un liposoluble de chargement correspondant à un taux $T_c$, on aura $T_i = t_i + T_c$. Dans le cas où les liposolubles associés au polymère kératinique sont de même nature que les liposolubles existant dans la matière première, dont est extrait le polymère kératinique, le dérivé kératinique résulte d'une délipidation totale ou partielle de la matière première utilisée et il est particulièrement intéressant pour absorber les lipides du sbstrat kératinique traité, notamment pour absorber le sébum de la peau ou des cheveux.

Dans une troisième variante, le dérivé kératinique est un polymère associé à un ou plusieurs liposoluble(s) à un taux $T_s$ supérieur à celui $T_n$ existant dans la matière première dont provient ledit polymère kératinique. Dans cette variante, on peut prévoir que les liposolubles associés au polymère kératinique comprennent, d'une part, ceux existant dans la matière première de départ à un taux $t_i$ inférieur ou égal à $T_n$ et, d'autre part, au moins un liposoluble de chargement à un taux $T_c$. On a: $T_s = t_i + T_c$. Les dérivés kératiniques sont, par exemple, obtenus par une délipidation de la matière première de départ suivie d'un chargement avec au moins un liposoluble de chargement. Le taux $t_i$ peut être nul ou voisin de zéro. Le taux $T_s$ est généralement inférieur à 70%.

Selon un premier mode de réalisation de cette troisième variante, le polymère kératinique est associé à au moins un liposoluble de chargement pris dans le groupe formé par ceux qui existent dans la matière première dont provient ledit polymère kératinique. Dans un autre mode de réalisation, le polymère kératinique est associé à au moins un liposoluble de chargement différent de ceux qui existent dans la matière première de départ. On peut prévoir que le taux global $T_c$ des liposolubles de chargement dans le dérivé kératinique selon l'invention est dû à un seul liposoluble de chargement.

De préférence, la matière première animale, d'où proviennent les chaînes kératiniques du dérivé, est choisie dans le groupe formé par la corne de sabot, notamment de cheval, les museaux et naseaux, notamment de bovins, la peau animale, notamment de porc, les cheveux et les plumes.

L'invention a aussi pour objet un procédé de préparation d'un dérivé kératinique tel que ci-dessus défini.

Lorsque le dérivé kératinique est sous-chargé en liposoluble(s) par rapport à la matière première de départ, le procédé selon l'invention, comporte toujours initialement une phase de délipidation.

Dans le cas où l'on veut préparer un dérivé kératinique surchargé en liposoluble(s) ou bien un dérivé kératinique comportant au moins un liposoluble n'existant pas dans la matière première de départ, le procédé selon l'invention comporte toujours une phase de chargement en liposoluble(s) de la matière kératinique d'origine animale.

Le procédé selon l'invention peut mettre en œuvre des matières kératiniques ayant subi, avant délipidation, un traitement chimique en vue de modifier la structure du polymère kératinique; dans une variante, le traitement chimique précité est réalisé sur la matière kératinique avant son chargement en liposoluble(s).

Selon un premier procédé, pour réaliser une délipidation initiale, on soumet la matière première, sous agitation, pendant un temps compris entre 20 min et 24 heures (ce temps dépendant du taux $t_i$ souhaité) à l'action de(s) solvant(s) pour les lipides qui s'y trouvent. Selon un mode préféré de mise en œuvre, le(s) solvant(s), qui permet la délipidation, est un mélange chloroforme/méthanol dans les proportions comprises entre 1/1 et 2/1 en volume, ou encore un mélange dichlorométhane/méthanol dans une proportion 3/1 en volume; on peut, avantageusement, prévoir que la matière première traitée par le(s) solvant(s) soit initialement broyée. Pour obtenir un polymère kératinique à partir de ce matériau, on réalise ensuite une purification de type connu par la succession d'étapes suivantes:

a) on lave l'extrait avec un tampon approprié;

b) on solubilise l'extrait lavé à un pH compris entre 8,5 et 9,5 dans un milieu solubilisant approprié;

c) on élimine les fractions insolubles et on dialyse la solution;

d) on précipite la kératine en amenant le pH entre 6 et 5,8;

e) éventuellement, on répète plusieurs fois l'ensemble des étapes b), c), et d) ci-dessus;

f) on récupère la kératine précipitée dans la phase d).

Si la purification est suffisante, on obtient ainsi un dérivé kératinique totalement hydrosoluble.

On peut, avantageusement, prévoir d'utiliser pour l'étape a) ci-dessus mentionnée un tampon à forte concentration de chlorure de potassium; le milieu solubilisant de l'étape b) est, avantageusement une solution aqueuse («T.U.M.E.») de trihydroxyméthylaminométhane formant un tampon à un pH de 8,9 et contenant de l'urée (8 M) et du mercapto-éthanol (0,2 M).

Dans le cadre de ce premier procédé, quand on veut effectuer un chargement en liposolubles, on peut le faire directement sur la matière première kératinique d'origine animale que l'on utilise. Mais, dans une autre variante, le chargement en liposolubles s'effectue sur une matière première ayant subi préalablement une phase de délipidation. La phase de chargement en liposolubles peut être effectuée en mettant en contact, sous agitation et pendant un temps compris entre 30 mn et 12 heures, selon la valeur que l'on veut donner à $T_c$, le matériau à charger en liposolubles, mis sous forme particulière, avant (ou après) purification et extraction du polymère kératinique, avec une solution d'au moins un liposoluble de chargement dans un solvant, puis en éliminant par évaporation ledit solvant. L'excès de liposoluble(s) de chargement peut être éliminé par lavages successifs répétés avec une solution de chlorure de sodium à 9‰; dans le cas où le matériau chargé a subi une phase de purification et d'extraction du polymère kératinique, on préfère enlever l'excès de liposoluble(s) par une solution aqueuse «T.U.M.E.» ci-dessus définie.

Cependant selon un deuxième procédé, la matière première kératinique est d'abord soumise, avant tout traitement affectant son chargement lipidique initial, à une extraction permettant d'obtenir une kératine native, notamment par la succession d'étapes a) à f) ci-dessus mentionnée; après quoi, on délipide la kératine native obtenue par un traitement en milieu solvant analogue à celui utilisé pour la délipidation dans le premier procédé; puis si l'on veut recharger la kératine en liposoluble(s), on apporte le(s) liposoluble(s) en milieu solvant, on forme le dérivé kératinique rechargé au cours d'une phase de solubilisation de la kératine et on précipite ce dérivé par variation du pH. Le dérivé est alors recueilli par centrifugation; l'excès de liposoluble(s) est éliminé avec le surnageant. Le culot peut être utilisé tel que (forme solide) ou resolubilisé et redialysé (forme solubilisée). L'association du matériel kératinique avec le(s) liposoluble(s) est réalisée dans le mélange «T.U.M.E.» précédemment défini.

On constate que le dérivé obtenu est un complexe formé grâce à une déstabilisation des α-hélices de la kératine suivie d'une ré-aggrégation; ce complexe est caractérisé par une constante d'affinité analogue à celle définie dans le modèle mathématique de Scatchard.

On a constaté que l'utilisation de dérivés kératiniques surchargés, sous-chargés ou rechargés en liposoluble(s) était bien tolérée par les substrats kératiniques, tels que la peau, les cheveux humains ou les cils. Cette bonne tolérance est, vraisemblablement, due au fait que les liposolubles apportés sur les substrats kératiniques sont associés à des chaînes kératiniques d'origine naturelle obtenues à partir d'une matière d'origine animale. Les chaînes kératiniques, qui apportent les liposolubles, assurent un effet de surface et ne pénètrent pas mais, par contre, libèrent lentement les liposolubles, ce qui explique à la fois une bonne tolérance vis-à-vis du substrat traité, un effet retard et l'efficacité du traitement.

La présente invention a donc, également, pour objet une composition de traitement d'un substrat kératinique, ladite composition contenant au moins un dérivé kératinique tel que ci-dessus défini. Au sens de la présente description, le mot «traitement» inclut le simple maquillage.

Ce dérivé kératinique peut être soit à l'état dissous, sout à l'état de suspension ou encore sous forme pulvérulente.

Il est clair qu'une même composition peut comporter une pluralité de dérivés kératiniques ayant, chacun, son action propre par la nature des liposolubles qu'il amène sur le substrat, ou par la quantité de liposolubles qu'il présente, l'action de la composition étant alors la résultante des actions unitaires des différents dérivés kératiniques utilisés. On a constaté que l'on obtenait des résultats particulièrement intéressants en utilisant, comme matière première pour les dérivés kératiniques, des matériaux kératiniques très solubles dans les tampons alcalins contenant de l'urée: c'est le cas, en particulier, des kératines extraites de la corne de sabots, notamment de cheval, des plumes, des cheveux et, plus particulièrement, du museau ou du naseau, notamment de bovin, ou de la peau, notamment de porc.

Lorsque le dérivé kératinique dans la composition selon l'invention est présent à l'état dissous, sa concentration est avantageusement comprise entre 0,1% et 10% en poids par rapport au poids total de la composition. Le pH de la composition est, de préférence, compris entre 6 et 10.

Lorsque le dérivé kératinique dans la composition selon l'invention est présent à l'état de suspension ou dispersé sous forme pulvérulente, sa concentration peut atteindre 70%.

La composition se présente sous forme de solution, d'émulsion, de crème, de gel, de suspension, de poudre.

On a constaté que les compositions selon l'invention permettaient d'obtenir des résultats cosmétiques très intéressants.

Lorsque la composition contient un dérivé kératinique partiellement ou totalement délipidé, elle peut être utilisée comme produit dégraissant pour la peau ou les cheveux gras: on peut, notamment, utiliser des dérivés kératiniques partiellement ou totalement délipidés selon l'invention dans des lotions dont l'application est suivie ou non d'un rinçage, telles que des lotions «après-shampooing» ou des lotions apaisantes «après-soleil», dans des shampooings, ou dans des crèmes, telles que des crèmes filtrantes solaires. Si la matière première de départ est une kératine contenant environ 10% de liposolubles, le dérivé kératinique de la composition peut être amené après la phase de délipidation, à contenir moins de 1‰ de liposolubles et ce dérivé se charge du sébum excédentaire qui existe sur le substrat où l'on applique la composition selon l'invention. On constate d'ailleurs, en appliquant en lotion un gel de kératine délipidée sur des cheveux naturels, une augmentation significative (+23%) de la mouillabilité des cheveux à l'acide oléique, ce qui montre bien l'action préalable «d'éponge ou buvard à sébum» de ce produit. Si l'on applique sur la peau d'une souris «hairless» une composition contenant un dérivé kératinique sensiblement délipidé, on constate une augmentation importante de la cohésivité (mesurée par desquamation forcée selon la méthode décrite par KERMICI, BODEREAU, AUBIN, J. SOC. COSMET. CHEM. 28, 151–164, 1977); une application journalière pendant 3 semaines d'une composition constituée par une solution aqueuse renfermant 1% en poids de dérivé(s) délipidé(s) a permis de noter une augmentation de 55% de la cohésivité. On a mis en évidence également une réduction de la perte insensible en eau transépidermique mesurée à l'aide d'un évaporimètre «Servometer EP 1».

On a également réalisé, selon l'invention, des compositions contenant des dérivés kératiniques obtenus en soumettant la matière première d'origine animale à une phase de délipidation suivie d'une phase de fixation d'un liposoluble de chargement.

Comme liposoluble de chargement, on a, à titre d'exemple, utilisé le cholestérol, des lécithines, de l'acide oléique, de la trioléine, de l'acide phosphatidique, du 3-benzylidène-camphre, ou de la vitamine F. A partir d'une matière première constituée par du stratum corneum de porc, après une phase de délipidation totale, on a réalisé, par exemple, une phase de relipidation, qui a permis d'obtenir un dérivé kératinique contenant un taux pondéral de 27% de lécithine. Un dérivé kératinique extrait de la corne de sabot de cheval, et sur lequel on avait fixé 60% de lécithine, en solution aqueuse à 1%, a été appliqué journellement sur des souris «hairless» pendant 3 semaines: après ce traitement, la cohésivité de la peau traitée, mesurée comme indiqué précédemment, a augmenté de 40%. Si l'on utilise, comme liposoluble de chargement, le cholestérol au lieu de la lécithine, on obtient, en pratiquant comme ci-dessus indiqué sur les souris «hairless», une amélioration de 50% de la cohésivité. On a noté, en outre, une réduction de 30% de la perte insensible en eau transépidermique. Si l'on compare l'action d'un dérivé kératinique totalement délipidé et celle d'un dérivé kératinique rechargé par la méthode ci-dessus indiquée, on constate, dès le deuxième jour, pour les kératines délipidées et les kératines délipidées enrichies en lécithines et, dès le sixième jour, pour les kératines délipidées enrichies en acide oléique, une réduction d'environ 25% de la perte insensible en eau. On a noté que les dérivés kératiniques rechargés après délipidation avec de l'acide oléique rendaient la peau moins déformable après plusieurs applications (propriétés astringentes) alors que, si le rechargement s'effectue avec des lécithines, la peau devient, au contraire, plus souple et plus élastique.

Dans tous les cas, on constate une amélioration sur le plan douceur et aspect de l'état cutané des animaux traités: ces paramètres ont été évalués par des observateurs entraînés utilisant une méthode de score d'appréciation (méthode en double aveugle).

Par ailleurs, on a testé, sur des cheveux naturels, l'action d'un dérivé kératinique obtenu à partir de stratum cornéum de porc après délipidation totale et rechargement avec un taux pondéral de 30% d'acide oléique ou de 30% de lécithine. Le traitement a été effectué en introduisant ce dérivé dans une composition de rinçage après shampooing. On constate que le coefficient de frottement à sec des cheveux traités est réduit de 15% par rapport au coefficient des cheveux non traités: le produit améliore donc la douceur des cheveux naturels et facilite leur démêlage.

En outre, les dérivés kératiniques ci-dessus cités, rechargés en acide oléique ou en lécithine, ont été étudiés quant à leurs propriétés d'étalement sur une couche cornée. On a constaté au moyen d'un microscope électronique à balayage que ces dérivés conduisaient à l'obtention de films homogènes continus. Ces produits présentent donc des propriétés filmogènes intéressantes. Une conséquence de ces propriétés est que, ayant un effet occlusif, ces dérivés favorisent la cicatrisation de la peau: on a testé cette propriété en créant sur un abdomen de cobaye une cicatrice épidermique, par succion et découpe du toit d'un blister, et en évaluant la cicatrisation 24 heures après application par mesure de la surface non ré-épidermisée.

Les résultats obtenus en utilisant une composition, qui contient un dérivé kératinique comportant du cholestérol comme lipide de chargement, peuvent également être obtenus en chargeant en cholestérol une matière première kératinique non-préalablement délipidée. On a pu, par exemple, prendre comme matière première de la corne de sabot de cheval contenant, à l'état naturel, 0,5% en poids de lipides, et charger cette matière première de 12% en poids de cholestérol.

Pour mieux faire comprendre l'objet de l'invention, on va en décrire maintenant, à titre d'exemples purement illustratifs et non limitatifs, plusieurs modes de réalisation et de mise en œuvre.

Exemple 1:
Préparation d'une kératine enrichie soluble
Première étape: obtention d'une matière première kératinique:

De la peau de porc, préalablement lavée à l'eau, est débarassée de son tissu adipeux par grattage. Des carrés de peau de 10 cm × 10 cm sont placés dans une enceinte saturée de vapeur d'ammoniaque pendant 30 min à température ambiante. Ces carrés sont, ensuite, incubés à 37° pendant une heure dans une solution de trypsine à 0,05% en milieu tampon tri-hydroxyméthylaminométhane/acide chlorhydrique $5 \times 10^{-2}$ M (pH = 7,9). Le stratum cornéum est récolté sous forme de feuillets, comme décrit dans l'article de FERGUSSON, BRIT. J. DERMATOL. 96, 21, 1977. Ces feuillets de stratum cornéum sont réduits en poudre par broyage dans l'azote liquide.

Deuxième étape: phase de délipidation:
La poudre de stratum cornéum obtenue est délipidée selon la méthode décrite par FOLCH (J. BIOL. CHEM. 226, 497, 1957).

La poudre obtenue dans la première étape est répartie en lots de 500 mg. Chaque lot est repris par 30 ml d'un mélange chloroforme-méthanol dans le rapport volumique 2/1. On homogénéise à 0°C; l'homogénat, après agitation, est filtré sur entonnoir de verre fritté puis rincé par 20 ml du mélange chloroforme-méthanol ci-dessus indiqué.

L'opération ci-dessus définie est généralement suffisante pour dégraisser entièrement le tissu; l'efficacité de la délipidation est vérifiée par l'analyse des lipides recueillis dans les filtrats. Si la délipidation n'a pas été suffisante, on peut reconduire l'opération plusieurs fois de suite. La poudre de stratum cornéum récupérée sur le filtre est finalement séchée sous vide.

Troisième étape: Phase de chargement en lipides:
500 mg de la poudre obtenue à la fin de la deuxième étape sont placés dans 20 ml d'une solution chloroforme-méthanol (rapport volumique 2/1) contenant 15% de lécithines (lécithines du jaune d'œuf d'origine MERCK, Réf. 5331); on maintient une agitation magnétique pendant 4 heures. Le solvant est, ensuite, évaporé dans un évaporateur «BRINKMAN» sous azote à 55°C. On obtient, ainsi, une poudre de stratum cornéum enrichie en phospholipides.

Quatrième étape: lavage de la kératine enrichie:
Les lots de stratum cornéum enrichis en lécithines obtenus à la fin de la troisième étape sont placés dans 50 ml de tampon phosphate 0,2 M (pH = 7,4). L'ensemble est maintenu sous agitation pendant une heure à température ambiante.

Après centrifugation, le culot est homogénéisé dans 30 ml d'une solution contenant:

- tampon tri-hydroxyméthylaminométhane/acide chlorhydrique: 10 mM (pH = 9)
  - chlorure de sodium: 20 mM
  - chlorure de potassium: 1,5 M

Les culots de matériau corné recueillis après centrifugation sont séchés. On a, ainsi, éliminé les protéines non kératiniques contaminantes et l'excès de lécithines non fixées. On obtient ainsi une kératine enrichie sous forme de poudre.

Cinquième étape:

On réalise une phase de solubilisation par le procédé suivant:

Les culots de matériau corné obtenus à la fin de la quatrième étape sont solubilisés dans 30 ml d'un mélange «T.U.M.E.» contenant:

- urée: 6 M
- mercapto-éthanol: 0,2 M
- tampon tri-hydroxyméthylaminométhane/acide chlorhydrique: 0,5 M (pH = 9).

Cette solubilisation s'effectue sous forte agitation à la température ambiante pendant 24 heures. Après centrifugation, les culots sont de nouveau soumis à une phase de solubilisation. On répète ainsi deux fois ce traitement. Les différents surnageants sont recueillis et dialysés pendant 48 heures contre 5 litres de tampon tri-hydroxyméthylaminométhane/acide chlorhydrique 0,05 M (pH = 9), avec trois changements, à température ambiante.

On purifie en abaissant le pH du dialysat obtenu dans la phase précédente jusqu'à 5,5 en ajoutant de l'acide chlorhydrique 0,1 N: on précipite ainsi le dérivé kératinique. Le précipité est resolubilisé dans 30 ml d'un mélange »T.U.M.E.» identique à celui utilisé précédemment. Après centrifugation, les surnageants correspondant aux différents lots traités sont dialysés contre un tampon tri-hydroxyméthylaminométhane/acide chlorhydrique 0,05 M (pH = 8) contenant un conservateur.

On obtient ainsi une solution aqueuse d'un dérivé kératinique selon l'invention chargé en lécithine, les chaînes kératiniques étant extraites de la peau de porc.

Contrôle du produit obtenu à l'exemple 1

On a contrôlé le produit obtenu à l'exemple 1 de la façon suivante:

1° – l'analyse électrophorétique en gel de polyacrylamide S.D.S. a permis à la fois d'authentifier les sous-unités caractéristiques de la kératine de porc et de vérifier l'absence de polypeptides contaminants.

2° – on a mis en évidence la présence dans les produits obtenus de lécithines. Pour ce faire, une partie des produits obtenus est lyophilisée et les lécithines en sont extraites par la méthode décrite dans la deuxième étape de cet exemple. Les lécithines extraites des produits obtenus sont visualisées par chromatographie sur couche mince dans un système de solvant chloroforme/méthanol/ammoniaque 6 N (rapports volumiques 65/30/5). La révélation du chromatogramme s'effectue par carbonisation à l'étuve après un bain d'acide sulfurique. Par ailleurs, les lécithines de l'extrait chloroforme-méthanol sont dosées par la méthode de CHABROL-CHARONNAT (Press Med. 45 (I), 713, 1937). Au cours de ce dosage utilisant le réactif sulfophosphovanillique, les lécithines sont dosées en se rapportant à une gamme étalon de lécithines. Le lipide fixé étant unique, cette évaluation revêt un caractère de spécificité.

On constate que les lécithines associées aux chaînes kératiniques dans le produit obtenu sont celles utilisées dans la troisième étape, la quantité de lécithines associées aux chaînes kératiniques étant d'environ 30% en poids par rapport au poids total du produit.

Exemples 2 à 6:

Dans une opération analogue à celle décrite dans l'exemple 1, on a pu fixer sur des kératines de stratum cornéum de proc préalablement délipidé le taux de lipides défini dans le tableau ci-dessous:

| N° Exemple | Lipides | | % de lipides/kératines | |
|---|---|---|---|---|
| 2 | Cholestérol | | 8 | |
| 3 | Acide oléique | | 22 | |
| 4 | Trioléine | | 27 | |
| 5 | Acide phosphatidique | | 18 | |
| 6 | Mélange | cholestérol | 58 | 13 |
| | | Acide oléique | | 16 |
| | | Trioléine | | 18 |
| | | Lécithines | | 11 |

Les quantités de lipides ont été estimées par dosages spécifiques biochimiques.

Exemple 7:

On solubilise 1 g de stratum cornéum de porc dans un milieu «T.U.M.E.» comme indiqué à l'exemple 1. On dialyse la solution contre un tampon à pH 9 comme indiqué à l'exemple 1. Après précipitation sélective à pH = 5,5 on recueille le culot de kératine par centrifugation et on procède à la délipidation dudit culot en utilisant le processus défini à la deuxième étape à l'exemple 1.

On reprend la kératine délipidée et on la met, pendant 12 heures, sous agitation en présence de 150 mg d'un filtre solaire constitué par du 3-benzylidène-camphre dans un milieu solvant organique (chloroforme/méthanol 2/1 en volume). Après élimination des solvants par évaporation, le complexe est extrait par solubilisation dans le milieu «T.U.M.E.» défini à l'exemple 1 puis dialysé et précipité au point isoélectrique de la kératine (pH = 5,5); l'excès de filtre est éliminé dans le surnageant.

Pour mesurer la quantité de filtre fixée sur la kératine, on procède alors à l'extraction du filtre liposoluble fixé au moyen d'un mélange chloroforme/méthanol (2/1 en volume) en agissant pendant 12 heures; le filtre passe dans la phase solvant. Le résultat obtenu est le suivant:

| | |
|---|---|
| Quantité de keratine extraite ........ | 405,5 mg |
| Quantité de filtre associé .......... | 42,6 mg |
| Filtre associé */kératine ............ | 10,5 % |
| Filtre associé */charge .............. | 28,4 % |

\* Le filtre a été dosé par absorption à 294 nm

Exemple 8:

1ère étape: Préparation d'un dérivé kératinique sulfonique

Dans un réacteur de deux filtres, on introduit 750 ml d'acide acétique pur et 350 g de kératine humide, à 30% de matière active, qui a été extraite de plumes de poulets par un mélange diméthylformamide/eau (2,5/1 en volume) au reflux pendant 8 heures.

L'ensemble est homogénéisé par agitation. On introduit en 40 min environ un mélange constitué de 375 ml d'eau oxygénée à 33% en poids et de 1125 ml d'acide acétique pur, cette introduction étant effectuée en refroidissant de façon permanente avec un bain de glace. On laisse ensuite revenir à température ambiante le mélange réactionnel et on maintient l'agitation pendant 15 heures. On dilue le mélange avec 5 litres d'eau. Le précipité obtenu est filtré et séché par lyophilisation. On obtient ainsi 70 g de poudre blanche.

2ème étape: Délipidation du dérivé kératinique

On réalise la délipidation du dérivé kératinique obtenu à la fin de la première étape au moyen d'un mélange chloroforme-méthanol (2/1 en volume) jusqu'à épuisement complet des lipides associés. On vérifie que la délipidation est totale par chromatographie en couche mince.

3ème étape: Relipidation du dérivé délipidé

On utilise la poudre blanche obtenue à la fin de la deuxième étape; on la met en contact avec un mélange de lipides (cholestérol-trioléine-acide oléique-lécithine) apporté en milieu chloroforme/méthanol (2/1 en volumes); le poids de chacun de ces lipides de chargement est égal à 15% du poids sec de la poudre blanche constituant le dérivé délipidé. On maintient le contact sous agitation pendant 12 heures. On élimine ensuite les solvants par évaporation puis on lave sous agitation pendant 4 heures avec un milieu riche en sel (chlorure de potassium: 1,5 M; chlorure de sodium; 0,25 M; trihydroxyméthylaminométhane: 0,25 M). On solubilise en milieu «T.U.M.E.» (défini à l'exemple 2) et ensuite, on précipite avec de l'acide chlorhydrique 0,1 M les dérivés kératiniques solubilisés; on centrifuge à 20 000 tours/min pendant 20 min. On élimine très soigneusement les surnageants qui contiennent l'excès de lipides et on sèche le culot de dérivé kératinique recueilli.

On vérifie sur le produit obtenu la fixation des lipides: pour ce faire, on reprend le produit par un mélange d'extraction chloroforme/méthanol (2/1 en volumes) et on extrait les lipides artificiellement fixés sur le dérivé kératinique aux fins d'analyse. Les résultats obtenus sont les suivants:

— Lipides associés au produit obtenu
à la fin de la 1ère étape　　　　　　　1%
— Lipides associés au produit obtenu
à la fin de la 2ème étape　　　　　　　0%
— Lipides associés au produit final relipidé:

| | | |
|---|---|---|
| — acide oléique | 7,5% | |
| — trioléine | 6% | |
| — cholestérol | 7,4% | 28,6% |
| — lécithine | 7,7% | |

Exemple 9

On solubilise 1 g de stratum cornéum de porc dans un milieu «T.U.M.E.», comme indiqué à l'exemple 1. On dialyse la solution contre un tampon à pH 9, comme indiqué à l'exemple 2. Après précipitation sélective à pH = 5,5, on recueille le culot de kératine par centrifugation et on procède à la délipidation des kératines à l'aide de 100 ml de chloroforme/méthanol (2/1 volume/volume). On reprend le culot de kératine délipidée et on le met pendant 12 heures sous agitation en présence de 150 mg de vitamine F en milieu chloroforme/méthanol (2/1 en volumes). Après élimination des solvants par évaporation, le complexe est extrait par solubilisation dans le milieu «T.U.M.E.» défini à l'exemple 1, puis dialysé et précipité au point isoélectrique de la kératine (pH = 5,5). Pour mesurer le taux de chargement, on procède alors à l'extraction de la vitamine F par agitation pendant 12 heures dans un mélange chloroforme/méthanol (2/1 en volumes); le mélange est ensuite filtré; on analyse le filtrat; on trouve que la kératine chargée contenait 25% en poids de vitamine F.

Exemple 10

On réalise une crème pour peaux sèches sous forme d'émulsion huile dans eau ayant la formulation suivante:

| | |
|---|---|
| — Stéarate de glycérol | 2,1 g |
| — Monostéarate de sorbitanne à 20 moles d'oxyde d'éthylène | 0,9 g |
| — Alcool cétylique | 0,5 g |
| — Alcool de lanoline | 3 g |
| — Acide stéarique | 2 g |
| — Huile de sésame | 10 g |
| — Huile d'arachide | 8 g |
| — Huile de pépins de raisin | 5 g |
| — Polymère carboxyvinylique | 0,3 g |
| — Triéthanolamine | 0,3 g |
| — Kératine enrichie en lécithine obtenue à l'exemple 1 | 3 g |
| — Parfum qs | |
| — Conservateur qs | |
| — Eau déminéralisée stérile qsp | 100 g |

Cette crème est appliquée sur le visage quotidiennement. La peau ne tire plus, est plus souple, plus douce.

Exemple 11

On prépare une composition de mascara répondant à la formulation suivante:

- Cire de Carnauba ............................ 16 g
- Kératine obtenue à la fin de
  l'exemple 8 .................................... 2 g
- Oléostéarate d'amino-2-propanediol-1,3 .... 12 g
- Hydroxy-éthyl-cellulose ...................... 1 g
- Eau déminéralisée ........................... 58,8 g
- Polysulfure d'aminosilicate .................. 8 g
- Oxyde de fer noir ............................ 2 g
- P-hydroxybenzoate de méthyle ............. 0,2 g

On note que l'utilisation de ce mascara apporte une bonne tenue du maquillage des cils au cours du temps, un bon confort et entraîne un effet de rallongement artificiel des cils.

Exemple 12

On prépare une crème antisolaire ayant la formulation suivante:

- Alcool cétyl stéarylique ...................... 2 g
- Monostéarate de glycérol .................... 4 g
- Alcool cétylique .............................. 4 g
- Huile de vaseline ............................ 5 g
- Stéarate de butyle ........................... 5 g
- Propylène glycol ............................. 7 g
- Huile de silicone ............................. 0,125 g
- Kératine obtenue selon l'exemple 7 ......... 7,5 g
- Conservateur ................................ 0,3 g
- Parfum ...................................... 0,4 g
- Eau qsp .................................... 100 g

On constate que cette crème appliquée sur la peau lui confère une bonne protection anti-solaire et une grande douceur. L'effet de protection présente une bonne rémanence malgré des bains fréquents.

Exemple 13

On réalise une émulsion pour maquillage fond de teint selon la formulation suivante:

- Acide lanolique ............................. 7,5 g
- Arginine .................................... 2,5 g
- Huile de jojoba ............................. 22,0 g
- Kératine préparée selon l'exemple 9 ........ 8 g
- Parahydroxybenzoate de propyle ........... 0,1 g
- Parfum ..................................... 0,2 g
- Colorants et pigments ...................... 10 g
- Eau déminéralisée stérile qsp .............. 100 g

On constate que ce fond de teint est très bien toléré par l'épiderme et lui confère de la douceur.

**Revendications**

1. Dérivé kératinique provenant d'une matière première contenant au moins une kératine d'origine animale, caractérisé par le fait qu'il est constitué par un polymère kératinique obtenu par séparation de substances kératiniques intracellulaires avec élimination des parois cellulaires, ledit polymère étant au moins partiellement délipidé.

2. Dérivé selon la revendication 1, caractérisé par le fait que le polymère kératinique au moins partiellement délipidé est associé à au moins un liposoluble, dont la quantité et/ou la nature est (ou sont) différente(s) de celle(s) des lipides associés à la kératine existant dans la matière première.

3. Dérivé selon l'une des revendications 1 ou 2, caractérisé par le fait que le polymère kératinique est totalement délipidé.

4. Dérivé kératinique selon l'une des revendications 1 à 3, caractérisé par le fait qu'il est constitué d'un polymère associé à au moins un liposoluble à un taux pondéral $T_i$ inférieur à celui $T_n$ existant dans la matière première dont provient le polymère kératinique.

5. Dérivé kératinique selon la revendication 4, caractérisé par le fait que les liposolubles associés au polymère kératinique sont de même nature que les lipides existant dans la matière première dont provient le polymère kératinique.

6. Dérivé kératinique selon la revendication 4, caractérisé par le fait que le(s) liposoluble(s) associé(s) au polymère kératinique compren(nent), d'une part, ceux qui existent dans la matière première de départ à un taux $t_i$ inférieur à $T_n$ et, d'autre part, au moins un liposoluble de chargement à un taux $T_c$ avec $T_i = t_i + T_c$.

7. Dérivé kératinique selon l'une des revendications 1 à 3, caractérisé par le fait qu'il est constitué d'un polymère associé à au moins un liposoluble à un taux pondéral $T_s$ supérieur à celui $T_n$ existant dans la matière première dont provient le polymère kératinique, le(s) liposoluble(s) associé(s) au polymère kératinique comprenant, d'une part, ceux qui existent dans la matière première de départ à un taux global $t_i$ tel que $0 \leqslant t_i \leqslant T_n$ et, d'autre part, au moins un liposoluble de chargement à un taux $T_c$ avec $T_s = t_i + T_c$.

8. Dérivé kératinique selon l'une des revendications 6 ou 7, caractérisé par le fait que le polymère kératinique est associé à au moins un liposoluble de chargement pris dans le groupe formé par les lipides qui existent dans la matière première dont provient le polymère kératinique.

9. Dérivé kératinique selon l'une des revendications 6 à 8, caractérisé par le fait que le polymère kératinique est associé à au moins un liposoluble de chargement différent de ceux qui existent dans la matière première dont provient le polymère kératinique.

10. Dérivé kératinique selon la revendication 9, caractérisé par le fait que l'un au moins des liposolubles de chargement est pris dans le groupe formé par les colorants liposolubles, les humectants liposolubles, les filtres solaires liposolubles, les stérols, les lipides phosphorylés, les acides gras, leurs esters, l'acide rétinoïque, les sphingolipides et les vitamines liposolubles.

11. Dérivé kératinique selon l'une des revendications 7 à 9, caractérisé par le fait que le taux global $T_c$ des liposolubles de chargement est dû à un seul liposoluble de chargement.

12. Dérivé kératinique selon l'une des revendications 7 à 10, caractérisé par le fait que le taux global $T_c$ des liposolubles de chargement est dû à un mélange de liposolubles de chargement.

13. Dérivé kératinique selon l'une des revendications 1 à 12, caractérisé par le fait que la matière première d'origine animale utilisée a subi, avant l'éventuel chargement en liposoluble(s), un traitement chimique modifiant le squelette kératinique.

14. Dérivé kératinique selon la revendication 13, caractérisé par le fait que le traitement chimique modifiant le squelette kératinique est une oxydation fournissant des groupes sulfoniques et/ou une hydrolyse et/ou une carboxyalkylation.

15. Dérivé kératinique selon l'une des revendications 1 à 13, caractérisé par le fait que la matière première, d'où provient le polymère kératinique, est choisie dans le groupe formé par la corne de sabot, notamment de cheval, les museaux et naseaux, notamment de bovins, la peau animale, notamment de porc, les cheveux et les plumes.

16. Composition de traitement d'un substrat kératinique, caractérisé par le fait qu'elle contient au moins un dérivé kératinique selon l'une des revendications 1 à 15.

17. Composition selon la revendication 16, caractérisée par le fait qu'elle contient, à l'état dissous, dans un support cosmétiquement acceptable, au moins un dérivé kératinique selon l'une quelconque des revendications 1 à 15 à une concentration comprise entre 0,1% et 10% en poids par rapport au poids total de la composition.

18. Composition selon l'une des revendications 16 ou 17, caractérisée par le fait que son pH est compris entre 6 et 10.

19. Composition selon l'une des revendications 16 à 18, caractérisée par le fait qu'elle se présente sous forme de solution, d'émulsion, de crème, de gel, de suspension ou de poudre.

20. Procédé de préparation d'un dérivé kératinique selon l'une des revendications 6 ou 7, caractérisé par le fait que l'on soumet une matière kératinique d'origine animale à une phase de chargement en liposoluble(s).

21. Procédé selon la revendication 20, caractérisé par le fait que la matière kératinique soumise à un chargement en liposoluble(s) est la matière première d'origine animale sans délipidation préalable.

22. Procédé selon la revendication 20, caractérisé par le fait que la matière première kératinique soumise à un chargement en liposoluble(s) est obtenue, à partir de la matière première d'origine animale, par une délipidation totale ou partielle.

23. Procédé selon l'une des revendications 20 à 22, caractérisé par le fait que la phase de chargement en liposoluble(s) s'effectue en mettant en contact, sous agitation et pendant un temps compris entre 30 min et 12 heures, d'une part, le matériau à charger avec, d'autre part, une solution d'au moins un liposoluble de chargement dans un solvant, puis en éliminant par évaporation ledit solvant.

24. Procédé selon la revendication 23, caractérisé par le fait que le matériau à charger en liposoluble(s) est la matière première d'origine animale mise sous forme particulaire, éventuellement après délipidation totale ou partielle.

25. Procédé selon la revendication 23, caractérisé par le fait que le matériau à charger en liposoluble(s) est une kératine native hydrosoluble extraite de la matière première d'origine animale.

26. Procédé selon la revendication 25, caractérisé par le fait que la kératine native hydrosoluble est soumise, avant chargement en liposoluble(s), à une délipidation totale ou partielle.

27. Procédé de préparation d'un dérivé kératinique selon la revendication 1, caractérisé par le fait que la matière première d'origine animale est soumise, avant tout traitement affectant son chargement lipidique initial, à une extraction permettant d'obtenir une kératine native.

28. Procédé selon la revendication 22, caractérisé par le fait que la matière kératinique subit, avant délipidation, un traitement chimique en vue de modifier la structure du polymère kératinique.

29. Procédé selon la revendication 20, caractérisé par le fait que la matière kératinique subit, avant chargement en liposoluble(s), un traitement chimique en vue de modifier la structure du polymère kératinique.

**Claims**

1. Keratin derivative originating from a raw material containing at least one keratin of animal origin, characterized in that it consists of a keratinic polymer obtained by isolation of intracellular keratinous substances with removal of the cell walls, the said polymer being at least partially delipidated.

2. Derivative according to Claim 1, characterized in that the at least partially delipidated keratinic polymer is associated with at least one liposoluble substance whose quantity and/or nature is (or are) different from that (those) of the lipids associated with the keratin existing in the raw material.

3. Derivative according to either of Claims 1 and 2, characterized in that the keratinic polymer is completely delipidated.

4. Keratin derivative according to one of Claims 1 to 3, characterized in that it consists of a polymer associated with at least one liposoluble substance in a weight ratio $R_l$ lower than that $R_n$ existing in the raw material from which the keratinic polymer originates.

5. Keratin derivative according to Claim 4, characterized in that the liposoluble substances associated with the keratinic polymer are of the same nature as the lipids existing in the raw material from which the keratinic polymer originates.

6. Keratin derivative according to Claim 4, characterized in that the liposoluble substance(s) associated with the keratinic polymer comprises (comprise), on the one hand, those which exist in the original raw material in a ratio $r_i$ lower than $R_n$ and, on the other hand, at least one liposoluble filler in a ratio $R_f$ where $R_i = r_i + R_f$.

7. Keratin derivative according to one of Claims 1 to 3, characterized in that it consists of a polymer associated with at least one liposoluble substance in a weight ratio $R_h$ higher than that $R_n$ existing in the raw material from which the keratinic polymer originates, the liposoluble substance(s) associated with the keratinic polymer comprising, on the one hand, those which exist in the original raw material in an overall ratio $r_i$ such that $0 \leqslant r_i \leqslant R_n$ and, on the other hand, at least one liposoluble filler in a ratio $R_f$ where $R_h = r_i + R_f$.

8. Keratin derivative according to either of Claims 6 and 7, characterized in that the keratinic polymer is associated with at least one liposoluble filler taken from the group consisting of the lipids which exist in the raw material which the keratinic polymer originates.

9. Keratin derivative according to one of Claims 6 to 8, characterized in that the keratinic polymer is associated with at least one liposoluble filler different from those which exist in the raw material from which the keratinic polymer originates.

10. Keratin derivative according to Claim 9, characterized in that at least one of the liposoluble fillers is taken from the group consisting of liposoluble dyes, liposoluble humectants, liposoluble sunscreens, sterols, phosphorylated lipids, fatty acids, their esters, retinoic acid, sphingolipids and liposoluble vitamins.

11. Keratin derivative according to one of Claims 7 to 9, characterized in that the overall ratio $R_f$ of the liposoluble fillers is due to a single liposoluble filler.

12. Keratin derivative according to one of Claims 1 to 10, characterized in that the overall ratio $R_f$ of the liposoluble fillers is due to a mixture of liposoluble fillers.

13. Keratin derivative according to one of Claims 1 to 12, characterized in that the animal starting raw material employed has been subjected to a chemical treatment modifying the keratin skeleton before being optionally filled with liposoluble substance(s).

14. Keratin derivative according to Claim 13, characterized in that the chemical treatment modifying the keratin skeleton is an oxidation supplying sulphonic groups and/or a hydrolysis and/or a carboxyalkylation.

15. Keratin derivative according to one of Claims 1 to 13, characterized in that the raw material from which the keratinic polymer originates is chosen from the group consisting of hoof horn, especially equine, muzzles and nostrils, especially bovine, animal skin, especially porcine, hair and feathers.

16. Composition for treating a keratinous substrate, characterized in that it contains at least one keratin derivative according to one of Claims 1 to 15.

17. Composition according to Claim 16, characterized in that it contains, in the form of solution in a cosmetically acceptable carrier, at least one keratin derivative according to any one of Claims 1 to 15 in concentration of between 0.1% and 10% by weight relative to the total weight of the composition.

18. Composition according to either of Claims 16 and 17, characterized in that its pH is between 6 and 10.

19. Composition according to one of Claims 16 to 18, characterized in that it is in the form of a solution, emulsion, cream, gel, suspension or powder.

20. Process for the preparation of a keratin derivative according to either of Claims 6 and 7, characterized in that a keratinous substance of animal origin is subjected to a stage of filling with liposoluble substance(s).

21. Process according to Claim 20, characterized in that the keratinous substance subjected to filling with liposoluble substance(s) is the raw material of animal origin without preliminary delipidation.

22. Process according to Claim 20, characterized in that the keratinous substance subjected to filling with liposoluble substance(s) is obtained from the raw material of animal origin by a complete or partial delipidation.

23. Process according to one of Claims 20 to 22, characterized in that the stage of filling with liposoluble substance(s) is performed by bringing together, with stirring and for a period of between 30 min. and 12 hours, on the one hand, the material to be filled with, on the other hand, a solution of at least one liposoluble filler in a solvent, and then removing the said solvent by evaporation.

24. Process according to Claim 23, characterized in that the material to be filled with liposoluble substance(s) is the raw material of animal origin put into particulate form, optionally after total or partial delipidation.

25. Process according to Claim 23, characterized in that the material to be filled with liposoluble substance(w) is a water-soluble natural keratin extracted from the raw material of animal origin.

26. Process according to Claim 25, characterized in that the water-soluble natural keratin is subjected to a complete or partial delipidation before filling with liposoluble substance(s).

27. Process for the preparation of a keratin derivative according to Claim 1, characterized in that the raw material of animal origin is subjected to an extraction permitting a natural keratin to be obtained, before any treatment affecting its initial lipid filling.

28. Process according to Claim 22, characterized in that the keratinous substance is subjected, before delipidation, to a chemical treatment with a view of modifying the structure of the keratinic polymer.

29. Process according to Claim 20, characterized in that the keratinous substance is subjected, before filling with liposoluble substance(s), to a chemical treatment with a view to modifying the structure of the keratinic polymer.

**Patentansprüche**

1. Keratinderivat, das aus einem Rohstoff stammt, der mindestens ein Keratin tierischen Ursprungs enthält, dadurch gekennzeichnet, daß es aus einem Keratinpolymer besteht, welches man durch Abtrennung intrazellularer Keratinsubstanzen unter Beseitigung von Zellwänden erhält, wobei das Polymer mindestens teilweise von Lipiden befreit ist.

2. Derivat nach Anspruch 1, dadurch gekennzeichnet, daß das mindestens teilweise von Lipiden befreite Keratinpolymer mit mindestens einer lipidlöslichen Substanz assoziiert ist, die sich hinsichtlich der Menge und/oder der Art von dem (den) Lipid(en) unterscheidet, das (die) mit dem im Rohstoff vorhandenen Keratin assoziiert ist (sind).

3. Derivat nach einem der Ansprüche 1 oder 2, dadurch gekennzeichnet, daß das Keratinpolymer vollkommen von Lipiden befreit ist.

4. Keratinderivat nach einem der Ansprüche 1 bis 3, dadurch gekennzeichnet, daß es aus einem Polymer besteht, das mit mindestens einer lipidlöslichen Substanz in einem Gewichtsanteil $T_i$ assoziiert ist, welcher niedriger ist als der Anteil $T_n$, der in dem Rohstoff vorliegt, von dem das Keratinpolymer stammt.

5. Keratinderivat nach Anspruch 4, dadurch gekennzeichnet, daß die mit dem Keratinpolymer assoziierten lipidlöslichen Substanzen die gleiche Natur besitzen wie die in dem Rohstoff, von dem das Keratinpolymer stammt, vorhandenen Lipide.

6. Keratinderivat nach Anspruch 4, dadurch gekennzeichnet, daß die lipidlösliche(n), mit dem Keratinpolymer assoziierte(n) Substanz(en) einerseits diejenigen, die im ursprünglichen Rohstoff in einem Anteil $t_i$ vorliegen, der niedriger ist als $T_n$, und andererseits mindestens eine lipidlösliche Beladung in einem Anteil $T_c$ mit $T_i = t_i + T_c$ umfaßt (umfassen).

7. Keratinderivat nach einem der Ansprüche 1 bis 3, dadurch gekennzeichnet, daß es aus einem Polymer besteht, das mit mindestens einer lipidlöslichen Substanz assoziiert ist in einem Gewichtsanteil $T_s$, der größer ist als der in dem Rohstoff, von dem das Keratinpolymer stammt, vorliegende Anteil $T_n$, wobei die lipidlösliche(n), mit dem Keratinpolymer assoziierte(n) Substanz(en) einerseits diejenigen, die im ursprünglichen Rohmaterial vorliegen, in einem Gesamtanteil $t_i$, wobei $0 \leqslant t_i \leqslant T_n$ ist, und andererseits mindestens eine lipidlösliche Beladung in einem Anteil $T_c$ mit $T_s = t_i + T_c$ umfaßt (umfassen).

8. Keratinderivat nach einem der Ansprüche 6 oder 7, dadurch gekennzeichnet, daß das Keratinpolymer mit mindestens einer lipidlöslichen Beladung assoziiert ist, die ausgewählt ist unter den Lipiden, die in dem Rohmaterial, von dem das Keratinpolymer stammt, vorliegen.

9. Keratinderivat nach einem der Ansprüche 6 bis 8, dadurch gekennzeichnet, daß das Keratinpolymer mit mindestens einer lipidlöslichen Beladung assoziiert ist, die sich von den Lipiden unterscheidet, die im Rohstoff, von dem das Keratinmaterial stammt, vorliegen.

10. Keratinderivat nach Anspruch 9, dadurch gekennzeichnet, daß mindestens eine der lipidlöslichen Beladungen ausgewählt ist unter lipidlöslichen Farbstoffen, lipidlöslichen feuchthaltenden Mitteln, lipidlöslichen Sonnenfiltern, Sterinen, phosphorylierten Lipiden, Fettsäuren und deren Estern, Retinsäure, Sphingolipiden und lipidlöslichen Vitaminen.

11. Keratinderivat nach einem der Ansprüche 7 bis 9, dadurch gekennzeichnet, daß der Gesamtanteil $T_c$ an lipidlöslichen Beladungen auf einer einzelnen lipidlöslichen Beladung beruht.

12. Keratinderivat nach einem der Ansprüche 7 bis 10, dadurch gekennzeichnet, daß der Gesamtanteil $T_c$ an lipidlöslichen Beladungen auf einer Mischung von lipidlöslichen Beladungen beruht.

13. Keratinderivat nach einem der Ansprüche 1 bis 12, dadurch gekennzeichnet, daß das verwendete Rohmaterial tierischen Ursprungs zur Modifizierung des Keratinskelettes chemisch behandelt worden ist, bevor es gegebenenfalls mit lipidlöslichen Zusätzen beladen wird.

14. Keratinderivat nach Anspruch 13, dadurch gekennzeichnet, daß die das Keratinskelett modifizierende chemische Behandlung eine Oxidation unter Bildung von Sulfonsäuregruppen und/oder eine Hydrolyse und/oder eine Carboxyalkylierung ist.

15. Keratinderivat nach einem der Ansprüche 1 bis 13, dadurch gekennzeichnet, daß der Rohstoff, von dem das Keratinpolymer stammt, ausgewählt ist aus Horn von Hufen, insbesondere von Pferden, Mäulern und Nüstern, insbesondere von Rindern, tierischer Haut, insbesondere von Schweinen, Haaren und Federn.

16. Mittel zur Behandlung eines Keratinsubstrats, dadurch gekennzeichnet, daß es mindestens ein Keratinderivat nach einem der Ansprüche 1 bis 15 enthält.

17. Mittel nach Anspruch 16, dadurch gekennzeichnet, daß es mindestens ein Keratinderivat nach einem der Ansprüche 1 bis 15 in einer Konzentration von 0,1 bis 10 Gew.-%, bezogen auf das Gesamtgewicht des Mittels, in gelöstem Zustand in einem kosmetisch verträglichen Träger enthält.

18. Mittel nach einem der Ansprüche 16 oder 17, dadurch gekennzeichnet, daß der pH zwischen 6 und 10 liegt.

19. Mittel nach einem der Ansprüche 16 bis 18, dadurch gekennzeichnet, daß es als Lösung, Emulsion, Crème, Gel, Suspension oder Puder vorliegt.

20. Verfahren zur Herstellung eines Keratinderivats nach einem der Ansprüche 6 oder 7, dadurch gekennzeichnet, daß man ein Keratinmaterial tierischen Ursprungs mit einer lipidlöslichen Substanz oder lipidlöslichen Substanzen belädt.

21. Verfahren nach Anspruch 20, dadurch gekennzeichnet, daß das mit lipidlöslichen Substanzen beladene Keratinmaterial der Rohstoff tierischen Ursprungs ist, der zuvor nicht von Lipiden befreit wurde.

22. Verfahren nach Anspruch 20, dadurch gekennzeichnet, daß das mit lipidlöslichen Substanzen beladene Keratinmaterial ausgehend vom Rohstoff tierischen Ursprungs erhalten wird, indem man es völlig oder teilweise von Lipiden befreit.

23. Verfahren nach einem der Ansprüche 20 bis 22, dadurch gekennzeichnet, daß man die Beladung mit lipidlöslichen Substanzen durchführt, indem man unter Rühren und während eines Zeitraums von 30 Minuten bis 12 Stunden das zu beladende Material mit einer Lösung mindestens einer lipidlöslichen Beladung in einem Lösungsmittel in Kontakt bringt und dann dieses Lösungsmittel verdampft.

24. Verfahren nach Anspruch 23, dadurch gekennzeichnet, daß das mit einer lipidlöslichen Substanz oder mit lipidlöslichen Substanzen zu beladende Material der gegebenenfalls teilweise oder völlig von Lipiden befreite Rohstoff tierischen Ursprungs in Teilchenform ist.

25. Verfahren nach Anspruch 23, dadurch gekennzeichnet, daß das mit einer lipidlöslichen Substanz oder lipidlöslichen Substanzen zu beladende Material ein wasserlösliches natives Keratin ist, das aus dem Rohstoff tierischen Ursprungs extrahiert worden ist.

26. Verfahren nach Anspruch 25, dadurch gekennzeichnet, daß das wasserlösliche native Keratin vor dem Beladen mit einer lipidlöslichen Substanz oder lipidlöslichen Substanzen völlig oder teilweise von Lipiden befreit wird.

27. Verfahren zur Herstellung eines Keratinderivats nach Anspruch 1, dadurch gekennzeichnet, daß man den Rohstoff tierischen Ursprungs extrahiert, um ein natives Keratin zu erhalten, bevor man eine Behandlung zum ersten Beladen mit Lipiden durchführt.

28. Verfahren nach Anspruch 22, dadurch gekennzeichnet, daß man das Keratinmaterial, bevor man es von Lipiden befreit, chemisch behandelt, um die Struktur des Keratinpolymers zu modifizieren.

29. Verfahren nach Anspruch 20, dadurch gekennzeichnet, daß man das Keratinmaterial vor dem Beladen mit einer lipidlöslichen Substanz oder lipidlöslichen Substanzen chemisch behandelt, um die Struktur des Keratinpolymers zu modifizieren.